# EUROPÄISCHE PATENTSCHRIFT

(11) **EP 0 992 475 B1**
(45) Veröffentlichungstag und Bekanntmachung des Hinweises auf die Patenterteilung: **26.03.2003**
(21) Anmeldenummer: 99118370.8
(22) Anmeldetag: 16.09.1999
(51) Int. Cl.: C07C 29/141, C07C 29/145, C07C 31/10, C07C 31/20, C07C 31/26, B01J 23/46, B01J 8/02

(54) **Verfahren zur Herstellung von Alkoholen durch katalytische Hydrierung von Aldehyden oder Ketonen**
Process for the preparation of alcohols by catalytic hydrogenation of aldehydes or ketones
Procédé de préparation d'alcools par hydrogénation catalytique des aldéhydes ou cétones

(30) Priorität: 28.09.1998 DE 19844325
(43) Veröffentlichungstag der Anmeldung: 12.04.2000
(73) Patentinhaber: Degussa AG, 40474 Düsseldorf (DE)
(72) Erfinder: Haas, Thomas, Dr., 60316 Frankfurt (DE); Jäger, Bernd, Dr., 64297 Darmstadt (DE); Sauer, Jörg, Dr., 63517 Rodenbach (DE); Vanheertum, Rudolf, Dr., 63796 Kahl (DE)

(56) Entgegenhaltungen:
- EP-A- 0 319 208
- EP-A- 0 803 488
- WO-A-98/57913
- DE-A- 3 726 195
- FR-A- 2 526 782
- US-A- 3 144 490
- US-A- 4 413 152
- US-A- 4 487 980
- US-A- 4 520 211
- US-A- 5 451 390
- US-A- 5 495 055
- DATABASE WPI Section Ch, Week 199101 Derwent Publications Ltd., London, GB; Class E17, AN 1991-003139 XP002133816 & JP 02 279643 A (MITSUBISHI PETROCHEMICAL CO LTD), 15. November 1990 (1990-11-15)

## Beschreibung

Die Erfindung betrifft ein Verfahren zur Herstellung von Alkoholen durch katalytische Hydrierung von Aldehyden oder Ketonen, wobei ein Ru-Trägerkatalysator verwendet wird. Der erfindungsgemäß zu verwendende Katalysator wird in geringerem Umfang desaktiviert und weist damit eine höhere Standzeit auf als für diesen Zweck bisher verwendete Ru-Trägerkatalysatoren.

Es ist bekannt, Aldehyde und Ketone durch katalytische Hydrierung in die entsprechenden Alkohole zu überführen. Zur Hydrierung von Aldehyden und Ketonen werden als Katalysatoren häufig Nickel-Trägerkatalysatoren oder Raney-Nickel verwendet. Ein Nachteil derartiger Katalysatoren ist das Ni-Leaching, wobei Ni in gelöster Form in das flüssige Reaktionsmedium gelangt. Dadurch wird die Aufarbeitung des Reaktionsgemischs erschwert, und das ausgelaugte Ni muß mit anderen Nebenprodukten entsorgt, etwa verbrannt, werden, wobei carcinogenes NiO zurückbleibt.

Um die genannten Probleme zu vermeiden, wurden trägergebundene Edelmetallkatalysatoren, insbesondere Ru-Katalysatoren, verwendet. B.J. Arena (Applied catalysis A 87 (1992) 219-229) lehrt, zur Hydrierung von Glucose zu Sorbitol Ru-Katalysatoren auf Aluminiumoxid zu verwenden. Ein Nachteil dieses Katalysators ist die durch Desaktivierung bedingte kurze Standzeit. Bei der Desaktivierung von Ru-Al₂O₃ werden nicht nur desaktivierende Komponenten, wie Eisen, Schwefel und Gluconsäure, auf dem Katalysator niedergeschlagen, sondern gleichzeitig ändern sich die Eigenschaften des Ru- und des Al₂O₃-Trägers, die sich unter anderem in einer Agglomeration des Ru und Erniedrigung der BET-Oberfläche des Al₂O₃ zeigen. Um die Desaktivierung zu vermindern, sind zusätzliche Reinigungsmaßnahmen der Einsatzstoffe und/oder häufige Regenerierung des Katalysators erforderlich, wodurch das Verfahren aufwendiger und/oder weniger wirtschaftlich wird.

Gemäß "Carbohydrates as Organic Raw Materials III", ed. by H. van Bekkum et al. (1996) 52-54 läßt sich Sorbitol aus Polysacchariden, wie Stärke, gewinnen, wobei durch Hydrierung in Gegenwart eines Ru-Trägerkatalysators mit H-USY-Zeolith als Träger gleichzeitig die Hydrolyse des Polysaccharids und Hydrierung der freigesetzten Glucose erfolgen. Der Träger wirkt als Säurekatalysator. Nach diesem Dokument werden ähnliche Ergebnisse erzielt, wenn man eine Kombination aus 5 % Ru auf Aktivkohle als Hydrierkatalysator und Zeolith-ZSM 5 als Säurekatalysator einsetzt. Hinweise über die Standzeit des Katalysators lassen sich diesem Dokument nicht entnehmen.

Hydroxypivalinaldehyd oder sein Dimeres lassen sich gemäß US-Patent 4,933,473 durch katalytische Hydrierung in Neopentylglykol überführen. Als Katalysator dient eine Kombination von Platin, Ruthenium und Wolfram in bestimmtem Mengenverhältnis. Diese katalytisch wirksame Metallkombination kann auch auf einen Träger aus der Reihe SiO₂, Al₂O₃, MgO, TiO₂, ZrO₂, Zeolithe, Kohlenstoff, Siliciumcarbid und Diatomeenerde zur Anwendung gelangen. Die Selektivität ist unter Verwendung von Pt/Ru/W-Aktivkohle am höchsten und sinkt in der Reihenfolge Al₂O₃, SiO₂, TiO₂ als Träger stark ab. Weder die Beispiele noch Vergleichsbeispiele betreffen die Verwendung eines Katalysators auf der Basis von Ru als einzigem Edelmetall auf einem oxidischen Träger.

Wie von den Erfindern dieser Anmeldung festgestellt wurde, sind Umsatz und Selektivität und insbesondere die Katalysatorstandzeit unter Verwendung von Ru-Aktivkohle in der gattungsgemäßen Hydrierung in vielen Fällen ungenügend; auf die Probleme, die sich beim Einsatz von Ru-Al₂O₃ ergeben, wurde bereits hingewiesen.

Di JP 02-279643 betrifft die katalytische Reduktion von Aceton zu Isopropanol, wobei Ru auf einem Träger, wie Aktivkohle, Aluminiumoxid oder Kieselsäure als Katalysator dient. Wesentlich ist die Anwesenheit von mindestens 50 % Isopropanol in der zu hydrierenden Aceton enthaltenden Lösung. Gemäß Beispiel 4 erfolgt die Hydrierung bei 50 °C in Gegenwart eines an Aktivkohle gebundenen Ru-Katalysators.

Die US-Patente 4,487,980, 4,413,152, 4,520,211 und die FR 25 26 782 A richten sich auf die Hydrierung von Kohlenhydraten unter Verwendung von Ru-Trägerkatalysatoren.

Das US-Patent 5,495,055 betrifft die Hydrierung von Aceton und Regenerierung des verwendeten Ru-Trägerkatalysators, wie Ru auf Aluminiumoxid.

Im Hydrierverfahren gemäß DE 37 26 195 werden Aldehyde und Ketone hydriert, wobei als Katalysator Ru auf Kieselsäure oder Aktivkohle, wobei letztere als Träger bevorzugt wird, eingesetzt wird.

Im Hydrierverfahren gemäß EP 0 803 488 erfolgt die Hydrierung in Gegenwart eines in situ gebildeten Ru-Trägerkatalysators. Ausweislich der Beispiele erfolgt die Hydrierung bei 180 - 200 °C.

Im Verfahren gemäß US-Patent 3,144,490 erfolgt die Hydrierung von Aldehyden in Gegenwart eines Ru-Trägerkatalysators,wie Ru auf Aktivkohle und elementarem Sauerstoff.

Aufgabe der vorliegenden Erfindung ist demgemäß die Bereitstellung eines verbesserten Verfahrens zur katalytischen Hydrierung von Aldehyden und Ketonen zu den entsprechenden Alkoholen. Die Verbesserung richtet sich auf die Erhöhung der Standzeit des zu verwendenden trägergebundenen Ru-Katalysators.

Gefunden wurde ein Verfahren zur Herstellung eines Alkohols durch katalytische Hydrierung des entsprechenden Aldehyds oder Ketons, ausgenommen 3-Hydroxypropionaldehyd und Kohlehydrate, oder Ketons in wäßriger oder organischer Lösung bei einer Temperatur von 20 bis 100 °C und einem H₂-Druck von 0,5 bis 30 MPa unter Verwendung eines trägergebundenen Rutheniumkatalysators, das dadurch gekennzeichnet ist, daß man als Katalysator Ruthenium auf einem Träger aus TiO₂, mit einem Ru-Gehalt von 0,1 bis 20 Gew.-% verwendet.

Die Unteransprüche richten sich auf bevorzugte Ausführungsformen.

Die vorteilhafte Verwendung eines Ru-Katalysators mit dem anspruchsgemäßen oxidischen Trägermaterialien, nämlich TiO₂, wurde bereits im Verfahren zur Herstellung von 1,3-Propandiol aus 3-Hydroxypropionaldehyd gemäß der noch nicht veröffentlichten DE-Patentanmeldung 197 37 190.6 erkannt. Die Verwendung dieser Katalysatoren ist aber, wie nun gefunden wurde, nicht auf die Hydrierung von 3-Hydroxypropionaldehyd beschränkt. Die Offenbarung der DE-Patentanmeldung 197 37 190.6 wird daher in die Offenbarung der vorliegenden Anmeldung in vollem Umfang einbezogen.

Erfindungsgemäß zu verwendende Ru-Katalysatoren auf oxidischen Trägern sind beispielsweise beschrieben in "Catalysts Supports and Supported Catalysts" von Alvin B. Stiles, Butterworth 1987, Kapitel 2 und 3. Besonders zweckmäßig kann die Beschichtung des oxidischen Trägers mittels der "incipient wetness Method" erfolgen - siehe "Preparation of Catalyst" ed. by B. Delmon, P.A. Jacobs, G. Poncald, Amsterdam Elsevier 1976, Seite 13.

Hierzu wird die Wasseraufnahmekapazität des Trägers bestimmt. Danach wird eine wäßrige Rutheniumchloridlösung mit einer Konzentration entsprechend der späteren Rutheniumbeschichtung hergestellt. Der Träger wird entsprechend der Wasseraufnahmekapazität mit wäßrigem Rutheniumchlorid beladen. Anschließend wird der beladene Träger, vorzugsweise bei 20 bis 100 °C, bei Normaldruck in inertgasatmosphäre, wie Neon, Helium, Argon oder Luft, getrocknet, mit Wasserstoff bei einer Temperatur von vorzugsweise 100 bis 500 °C in einer Zeit von 20 Min. bis 24 Std. unter Verwendung eines 1 bis 100 Vol.-% Wasserstoff enthaltenden H₂/N₂-Gasgemischs reduziert und gegebenfalls chloridfrei, vorzugsweise < 100 ppm Cl⁻, gewaschen.

Gemäß einer bevorzugten wird ein pyrogen hergestelltes TiO₂, insbesondere ein durch Flammenhydrolyse hergestelltes TiO₂ als Träger eingesetzt.

Beispielsweise kann man ein durch Flammenhydrolyse aus Titantetrachlorid gewonnenes pyrogenes Titandioxid mit einer BET-Oberfläche von 40 bis 60 m²/g und einem Gesamtporenvolumen von 0,25 bis 0,75 ml/g verwenden, das eine mittlere Größe der Primärteilchen von 20 nm, eine Dichte von 3,7 g/cm³ und eine Röntgenstruktur aus 20 bis 40 % Rutil und 80 bis 60 % Anatas aufweist und dessen Verunreinigungen an Siliciumdioxid, Aluminiumoxid und Eisenoxid unter 0,5 Gew.-% liegen. Pyrogenes Titanoxid wie das Material P25 von Degussa ist als Träger für die katalytisch aktive Komponente besonders geeignet. Es weist eine hohe spezifische Oberfläche nach BET von im Mittel 50 m²/g (gemessen nach DIN 66131) auf.

Die Ru-Belegung des Trägers liegt im Bereich von 0,1 bis 20 Gew.-%, vorzugsweise 0,5 bis 10 Gew.-% und besonders bevorzugt 1 bis 5 Gew.-%.

Die Durchführung der Hydrierung kann in üblicher Weise, diskontinuierlich oder kontinuierlich erfolgen. Hierbei kann der Katalysator im flüssigen Reaktionsmedium suspendiert sein. Alternativ wird der Katalysator in Form von Formkörpern, wie Pellets, Granulat, Kugeln, Strangpreßlingen, eingesetzt und in einem Reaktor als Festbett angeordnet. Dieser Festbettreaktor kann in geflutetem Zustand als Blasenreaktor betrieben werden, vorzugsweise wird er aber als Rieselbettreaktor betrieben.

Die Druck- und Temperaturbedingungen wird der Fachmann an das zu hydrierende Substrat anpassen. Es ist ein Vorteil der erfindungsgemäß zu verwendenden Katalysatoren, daß ihre hohe Aktivität meistens milde Reaktionsbedingungen ermöglicht, beispielsweise etwa 20 bis 100 °C und 1 bis 10 MPa, insbesondere 2 bis 5 MPa H₂-Druck.

Die zu hydrierenden Aldehyde und Ketone können beliebige Struktur haben und aliphatisch, aromatisch, heteroaromatisch, aliphatisch-aromatisch oder aliphatischheteroaromatisch sein. Sie können auch andere funktionelle Gruppen enthalten - in diesem Fall ist vorher zu prüfen, ob diese funktionellen Gruppen unverändert bleiben sollen oder selbst hydriert werden sollen. Gemäß einer bevorzugten Ausführungsform werden eine oder mehrere Hydroxylgruppen enthaltende Carbonylverbindungen, ausgenommen Kohlenhydrate, in Polyole überführt.

Das Substrat kann, wenn es flüssig ist, per se oder in einem Lösungsmittel gelöst hydriert werden. Lösungsmittel können organisch oder wäßrig sein oder Gemische darstellen. Besonders bevorzugt wird Wasser als Lösungsmittel, dem bei Bedarf noch organische Lösungsvermittler zugegeben werden können.

Es ist bekannt, daß bei der Reduktion von Aldehyden und Ketonen Acetale und Ketale entstehen können. Um diese Nebenprodukte in situ zu spalten und vollständig in den gewünschten Alkohol zu überführen, ist es zweckmäßig, die Hydrierung in Gegenwart eines sauren Katalysators durchzuführen. Dieser Katalysator kann gelöst sein - beispielsweise im Falle einer Mineralsäure - oder ungelöst als Feststoffsäure vorliegen. Bei erhöhter Reaktionstemperatur wirkt der Träger TiO₂ selbst bereits als Säure.

Die erfindungsgemäß zu verwendenden, an dem anspruchsgemäßen TiO₂-Träger gebundenen Ru-Katalysatoren weisen eine überraschend hohe Standzeit auf, da sie viel langsamer desaktivieren als bisher verwendete trägergebundene Ru-Katalysatoren. Damit wird die Wirtschaftlichkeit des Verfahrens verbessert, da die Aktivität lang erhalten bleibt und Stillstandzeiten für die Katalysatorregenerierung minimiert und die Raum-Zeit-Ausbeute erhöht werden.

Die nachfolgenden Beispiele und Vergleichsbeispiele verdeutlichen den erfindungsgemäßen Effekt.

### Allgemeine Arbeitsvorschrift:

Die Katalysatoren wurden unter stationären Bedingungen getestet, um auch Aussagen über das Langzeitverhalten machen zu können. Die Hydrierung wurde kontinuierlich in einer Rieselbettanlage mit 140 ml Reaktorvolumen durchgeführt. Die Anlage umfaßte eine Flüssigkeitsvorlage, den Reaktor und einen Flüssigkeitsabscheider. Die Reaktionstemperatur wurde über einen Wärmeträgerkreislauf eingestellt. Druck und Wasserstoffstrom wurden elektronisch geregelt. Die wäßrige Lösung des Substrats (= Aldehyd oder Keton) wurde dem Wasserstoffstrom mit einer Pumpe zudosiert und das Gemisch am Kopf des Reaktors aufgegeben (Rieselbett-Fahrweise). Nach Durchlaufen des Reaktors wurde das gebildete Produkt in regelmäßigen Abständen aus dem Abscheider entnommen. Die Konzentration des Aldehyds beziehungsweise Ketons in der Eduktlösung, die Temperatur, der H₂-Druck und die Flüssigkeitsbelastung LHSV 1 h⁻¹ sind den nachfolgenden Beispielen zu entnehmen. In den Tabellen 1 und 2 sind die Ergebnisse der Versuche zusammengefaßt.

### Beispiele (B) 1 bis 2 und Vergleichsbeispiele (VB) 1 bis 3

Die Beispiele 1 und 2 nutzen zwar die erfindungsgemäß zu verwendenden Ru-Trägerkatalysatoren, allerdings beziehen sie sich, wie auch die Vergleichsbeispiele 1 bis 3, auf die Hydrierung von 3-Hydroxypropionaldehyd zu 1,3-Propandiol, welche im Hinblick auf die noch nicht veröffentlichte DE 197 37 190.6 nicht beansprucht wird. Die Reaktionstemperatur betrug 40 °C, der H₂-Druck 4 MPa, die Aldehyd-Konzentration der wäßrigen Lösung 10 Gew.-% und der LHSV-Wert 1 h⁻¹. Aus Tabelle 1 wird die überraschende Wirksamkeit der erfindungsgemäß zu verwendenden Katalysatoren - meist höherer Umsatz, insbesondere aber bessere Langzeitstabilität - im Vergleich zu den in den Vergleichsbeispielen eingesetzten nicht-erfinderischen Katalysatoren deutlich.

**Tabelle 1**

| Nr. | Katalysator | Betriebsdauer (h) | Umsatz (%) | Umsatzabnahme (%/h) |
|---|---|---|---|---|
| B 1.1 | 5 % Ru auf TiO₂ (P 25 der Degussa AG; Strangpreßlinge d = 1 mm) | 19 | 84 | |
| B 1.2 | " | 233 | 84 | 0 |
| B 2.1 | 5 % Ru auf SiO₂ (Silicagel V 432 der Fa. Grace d = 0,8 - 1,2 mm) | 48 | 90 | |
| B 2.2 | " | 434 | 89 | 0,26 |
| VB 1.1 | 10 % Ru auf Al₂O₃ (Speralite 521 der Fa. Rhone-Poulenc; d = 1,1 - 1,3 mm) | 72 | 79 | |
| VB 1.2 | " | 240 | 77 | 1,19 |
| VB 2.1 | 5 % Ru auf Aktivkohle (Norit ROX; d = 0,8 mm) | 24 | 99,7 | |
| VB 2.2 | " | 96 | 60 | 55,13 |
| VB 3.1 | 2 % Pt auf TiO₂ (P 25 der Degussa AG; d = 1 mm) | 20 | 60 | |
| VB 3.2 | " | 300 | 45 | 5,36 |

### Beispiele (B) 3 bis 6 und Vergleichsbeispiele (VB) 4 bis 7

Tabelle 2 zeigt die Ergebnisse - Umsatz und Selektivität - der Hydrierung verschiedener Edukte in Abhängigkeit von der Betriebsdauer und Katalysator; enthalten sind ferner die Reaktionstemperatur (T) und der LHSV-Wert. Die Konzentration des Edukts in Wasser betrug 10 Gew.-%, der H₂-Druck 4 MPa.

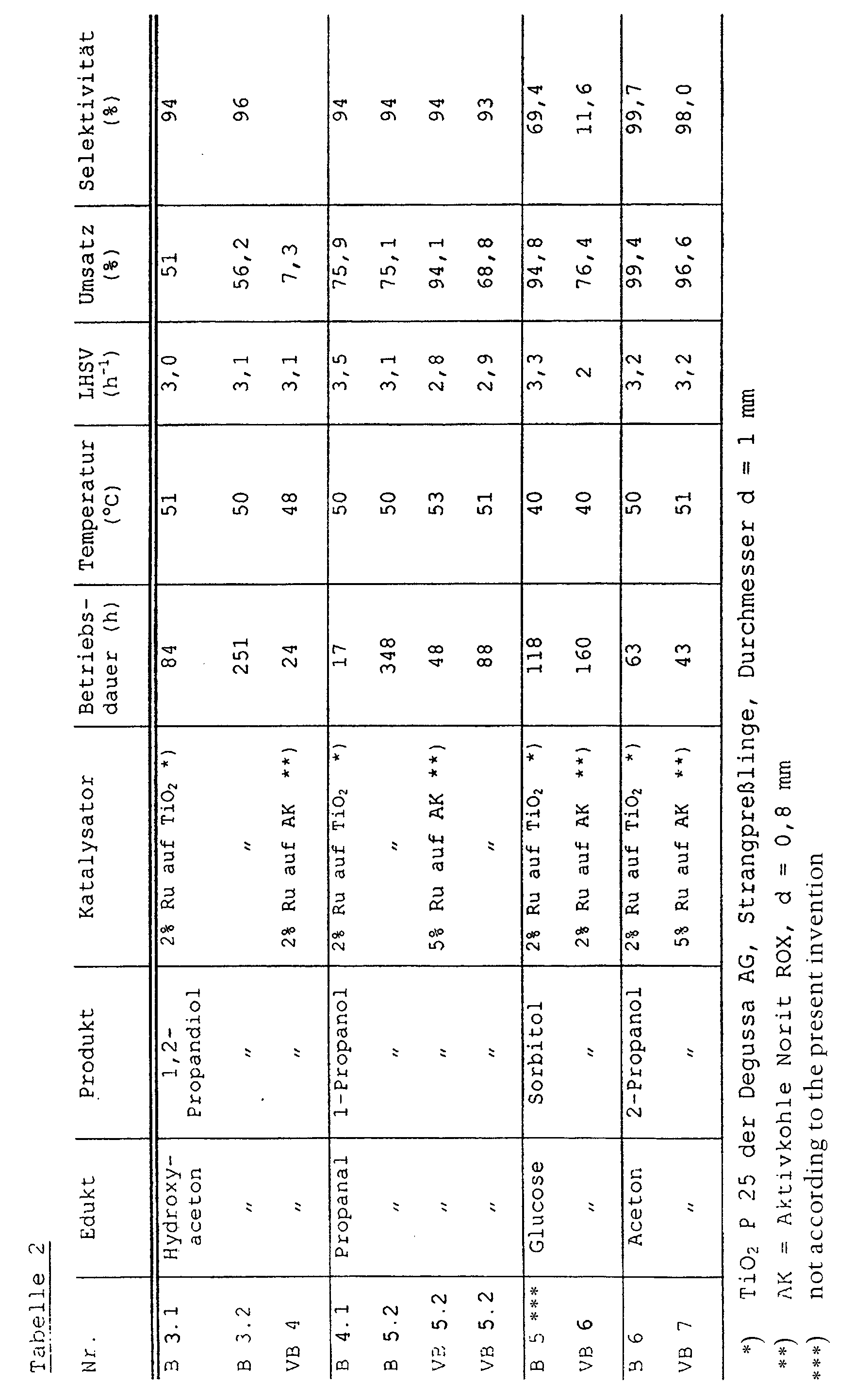

Unter Verwendung der Ru-Katalysatoren auf TiO₂ werden gegenüber Ru auf Aktivkohle nach einigen Stunden Betriebsdauer meistens höhere Umsätze erzielt. Wesentlich ist, daß diese hohen Umsätze und Selektivitäten auch nach langer Betriebsdauer im wesentlichen aufrechterhalten werden.

## Patentansprüche

1. Verfahren zur Herstellung eines Alkohols durch katalytische Hydrierung des entsprechenden Aldehyds oder Ketons, ausgenommen 3-Hydroxypropionaldehyd und Kohlenhydrate, in wäßriger oder organischer Lösung bei einer Temperatur von 20 bis 100 °C und einem H₂-Druck von 0,5 bis 30 MPa unter Verwendung eines trägergebundenen Rutheniumkatalysators,
**dadurch gekennzeichnet,**
**daß** man als Katalysator Ruthenium auf einem Träger aus TiO₂, mit einem Ru-Gehalt von 0,1 bis 20 Gew.-% verwendet.

2. Verfahren nach Anspruch 1,
**dadurch gekennzeichnet,**
**daß** man als Ru-Trägerkatalysator Ru auf einem pyrogen, insbesondere durch Flammenhydrolyse hergestellten TiO₂ verwendet.

3. Verfahren einem der Ansprüche 1 oder 2,
**dadurch gekennzeichnet,**
**daß** man zwecks Spaltung von anwesenden und/oder gebildeten Acetalen oder Ketalen die Hydrierung in Gegenwart eines homogenen oder heterogenen sauren Katalysators durchführt.

4. Verfahren nach einem der Ansprüche 1 bis 3,
**dadurch gekennzeichnet,**
**daß** man die Hydrierung in Form einer Rieselbett-Hydrierung durchführt.

## Claims

1. Process for the production of an alcohol by catalytic hydrogenation of the corresponding aldehyde or ketone, except 3-hydroxypropionaldehyde and carbohydrates, in aqueous or organic solution at a temperature of 20 to 100°C and a H₂ pressure of 0.5 to 30 mPa using a ruthenium catalyst bound to a support,
**characterised in that**
ruthenium on a support of TiO₂, with an Ru content of 0.1 to 20 wt.% is used as the catalyst.

2. Process according to claim 1.
**characterised in that**
Ru on a pyrogenic TiO₂, produced in particular by flame hydrolysis, is used as the Ru supported catalyst.

3. Process according to claim 1 or 2
**characterised in that**
in order to split acetals or ketals present and/or formed, hydrogenation is carried out in the presence of a homogeneous or heterogeneous acid catalyst.

4. Process according to one of claims 1 to 3,
**characterised in that**
hydrogenation is carried out in the form of trickle-bed hydrogenation.

## Revendications

1. Procédé de préparation d'un alcool par hydrogénation catalytique de l'aldéhyde correspondant ou de la cétone correspondante, à l'exception du 3-hydroxypropionaldéhyde et des hydrates de carbone, en solution aqueuse ou organique à une température de 20 à 100°C et une pression en H₂ de 0,5 à 30 MPa en utilisant un catalyseur de ruthénium fixé sur un support, **caractérisé en ce qu'**on utilise comme catalyseur du ruthénium sur un support en TïO₂, présentant une teneur en Ru de 0,1 à 20 % en poids.

2. Procédé selon la revendication 1, **caractérisé en ce qu'**on utilise comme catalyseur supporté de Ru du Ru sur du TiO₂ préparé par voie pyrogène, en particulier par hydrolyse de flamme.

3. Procédé selon l'une quelconque des revendications 1 ou 2, **caractérisé en ce qu'**on réalise l'hydrogénation en présence d'un catalyseur acide homogène ou hétérogène en vue de la dissociation des acétals ou des cétals présents et/ou formés.

4. Procédé selon l'une quelconque des revendications 1 à 3, **caractérisé en ce qu'**on réalise l'hydrogénation sous forme d'une hydrogénation en lit ruisselant.
